(19) Europäisches Patentamt
European Patent Office
Office européen des brevets

(11) Publication number : 0 472 392 A2

(12) # EUROPEAN PATENT APPLICATION

(21) Application number : 91307624.6

(22) Date of filing : 19.08.91

(51) Int. Cl.$^5$ : C07D 249/08, C07D 303/12, C07D 405/06, A61K 31/41

(30) Priority : 24.08.90 JP 223894/90

(43) Date of publication of application : 26.02.92 Bulletin 92/09

(84) Designated Contracting States : AT BE CH DE DK ES FR GB GR IT LI LU NL SE

(71) Applicant : IMPERIAL CHEMICAL INDUSTRIES PLC
Imperial Chemical House, Millbank
London SW1P 3JF (GB)

(71) Applicant : MOCHIDA PHARMACEUTICAL CO., LTD.
7, Yotsuya 1-chome
Shinjuku-ku Tokyo 160 (JP)

(72) Inventor : Murakami, Kimihiro
169-2, Subashiri, Oyama-cho
Suntou-Gun, Shizuoka-ken 410-14 (JP)
Inventor : Mochizuki, Hidenori
1409-52 Kamado
Gotemba-shi, Shizuoka-ken 412 (JP)

(74) Representative : Atkinson, John David et al
Imperial Chemical Industries PLC Legal Department: Patents PO Box 6 Bessemer Road
Welwyn Garden City Hertfordshire, AL7 1HD (GB)

(54) **Optically active triazole derivatives and compositions.**

(57) The compound (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol, which has antifungal activity, and is valuable in treatment of fungal infections in man and in other animals, pharmacologically acceptable salts thereof, solvates thereof and solvates of salts thereof, and also methods for its preparation, and medicinal compositions characterised in that they contain it as active ingredient. The invention also concerns intermediates for the preparation of the compound shown by the formula ((+)-I) and also methods for their preparation.

EP 0 472 392 A2

The present invention concerns (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol shown by the formula ((+)-I)

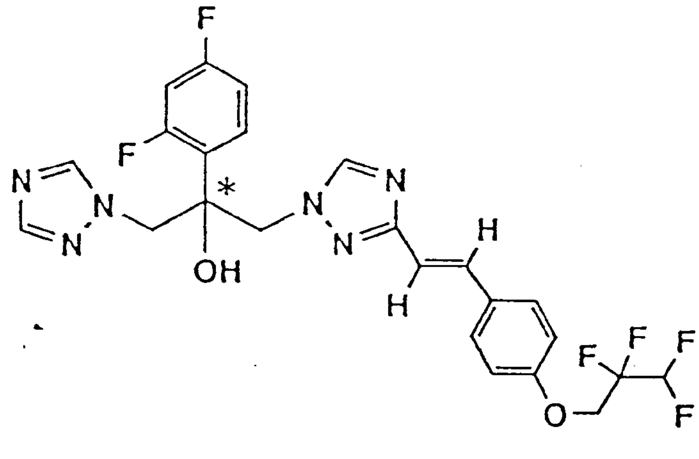

((+)-I)

(where * indicates an optically active centre), which has antifungal activity, and is valuable in treatment of fungal infections in man and in other animals, pharmacologically acceptable salts thereof, solvates thereof and solvates of salts thereof, and also methods for its preparation, and medicinal compositions characterised in that they contain it as active ingredient. The present invention also concerns intermediates for the preparation of the compound shown by the formula ((+)-I) and also methods for their preparation.
2-(2,4-difluorophenyl)-1-[3-[(E)-4-(2,2,3,3-tetrafluoropropoxy)-styryl]-1H-1,2,4-triazol-1-yl]-3-(1H-1,2,4-triazol -1-yl)propan-2-ol shown by the formula ((±-I)

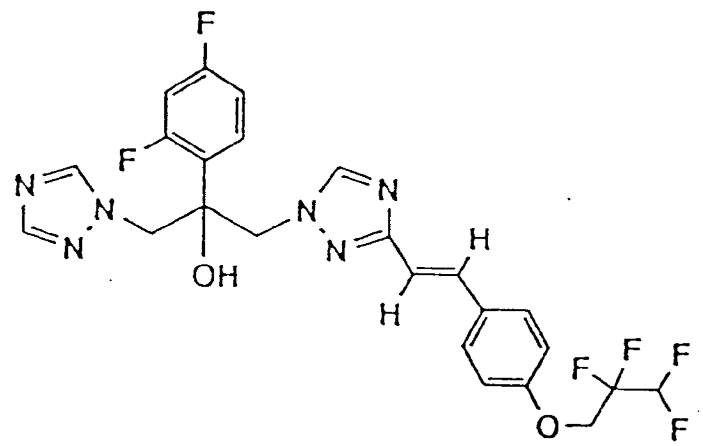

((±)-I

(hereinafter referred to as compound A) is known to possess an extremely high antifungal activity (Y H Thong et al., Journal of Antimicrobial Chemotherapy 21, 755-763,1988). Although compound A has an asymmetric carbon in the molecule, since disclosure of its structure in European Patent 0174769 it has only been dealt with as the racemic form. However, as can be seen from the lessons of the thalidomide affair, in compounds having an asymmetric carbon even if one of the optical isomers is a valuable compound it is possible for the racemate or the other optical isomer to have unexpected toxicity. Furthermore, cases are also known in which one optical isomer may surpass the opposite optical isomer in pharmacological activity and its kinetics in the body may be different. For reasons such as these, nowadays if racemates of compounds having an asymmetric carbon are developed, it has come to be required that detailed information be submitted concerning the pharmacological activity, toxicity, pharmacokinetics, etc, not only of the racemic form, but also of each optical isomer.

As a result of pharmacolological testing performed on compound A, the present inventors have confirmed that compound A certainly possesses strong antifungal activity. Further, as a result of repeated oral toxicity studies in monkeys, the present inventors confirmed that compound A has side-effects such as, at doses of 3 mg/kg/day or more, a decrease in serum cortisol level (histologically, slight to severe hyperplasia and increase of lipid droplets of the zona fasciculata in the adrenal cortex, and atrophy of the seminiferous tubules in the testes), and at doses of 30 mg/kg/day or more, prolongation of the QTc interval in the electrocardiogram (this

means a retardation of repolarisation and there is a risk that this could lead to serious arrhythmias). Furthermore, it was revealed by pharmacokinetic studies that compound A accumulates.

Consequently, in order actually to develop this compound as a pharmaceutical, how in any way to make it into a safe medicine constituted a considerable problem.

The present inventors, noting that compound A is a racemate, as an approach to the solution of the problems firstly thought of investigating the toxicity and pharmacological activity of each of its optical isomers. However, the optical isomers of compound A had not previously been isolated or prepared and nothing was known of their physical, chemical or pharmacological properties.

Accordingly, the present inventors turned their attention to the asymmetric carbon in compound A, and performed diligent research into the asymmetric synthesis of the optical isomers of compound A. As a result, they firstly succeeded in the stereoselective synthesis of the (-)- or (+)-2-(2,4-difluorophenyl)-propane derivatives shown by the formula (II)

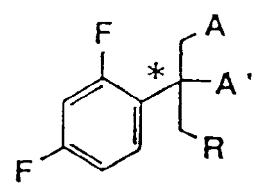

(II)

where * indicates an optically active centre, A and A′ together are an oxygen atom, or A′ is a hydroxy group and A is a hydroxy group, methanesulphonyloxy group or p-toluenesulphonyloxy group, and R is a hydroxy group, acetoxy group, 1H-1,2,4-triazol-1-yl group or 3-[(E)-4-(2,2,3,3-tetrafluoropropoxy)styryl]-1H-1,2,4-triazol-1-yl group, providing that both A and R are not simultaneously hydroxy groups. Next they discovered that it is possible to use each of these (-) and (+) optically active compounds as intermediates in the preparation of the compound of the present invention, shown by the formula ((+)-I), and also that it is possible to prepare the compound of the present invention, of formula ((+)-I), industrially using them.

Moreover optically active compounds stated to be the (+)-form or the (-)-form in the detailed description of the present invention are the compounds which show respectively (+) or (-) rotatory power with respect to the sodium D line, and are taken to be mutually enantiomerically related. Also, as regards the representation of compounds in the detailed description of the present invention, hereinafter a compound shown by the formula (N) is also represented as the compound N (N corresponds to the Roman numerals I-XX).

Thus we synthesized each optical isomer of compound A, and, firstly, as a result of investigating the antifungal activity of the (+)-form of compound A (compound (+)-I) and the (-)form (compound (-)-I), it became clear that compound (+)-I had remarkably powerful antifungal activity compared to compound (-)-I. Next, we tested the toxicity of compound (+)-I by repeated oral administration to monkeys

The result was that at a dosage of 10 mg/kg/day the compound (+)-I form did display a slight hyperplasia and increase of lipid droplets in the zona fasciculata in the adrenal cortex, but nonetheless even at a dosage of 30 mg/kg/day it did not display cardiotoxicity (prolongation of the QTc interval), testicular toxicity (atrophy of the seminiferous tubules) or decrease in serum cortisol; hence the present inventors discovered that in the case of compound (+)-I the antifungal activity and the side-effects are clearly separated. In addition, from the variation of the concentration in the plasma with time, it became clear that compound (+)-I does not display accumulation. Consequently, it was confirmed that compound (+)-I is capable of becoming a safe and highly valuable antifungal drug.

On the basis of the above results, in order clearly to separate the side-effects and the pharmacological activity of compound A, we synthesized its (+)-optical isomer (compound (+)-I) stereoselectively, realised that it would be good to continue its further development into a pharmaceutical, and hence accomplished the present invention.

The compound (+)-I of the present invention can be made into salts with pharmacologically acceptable inorganic acids or organic acids. As examples of these salts, salts with inorganic acids such as the hydrochloride, sulphate, and nitrate, salts with organic acids such as the acetate, p-toluenesulphonate, methanesulphonate and maleate, and amino acid salts such as the lysine salt and the arginine salt may be mentioned. The aforesaid salts are obtained by normal methods, for example by mixing solutions containing equimolar amounts of free compound (+)-I and the desired acid, and isolation of the desired salt by filtration (if insoluble) or by evaporation of the solvent.

The preparation of the intermediates according to the present invention and the preparation of compound (+)-I of the present invention using them as starting materials can for example be performed by a process such as the following:

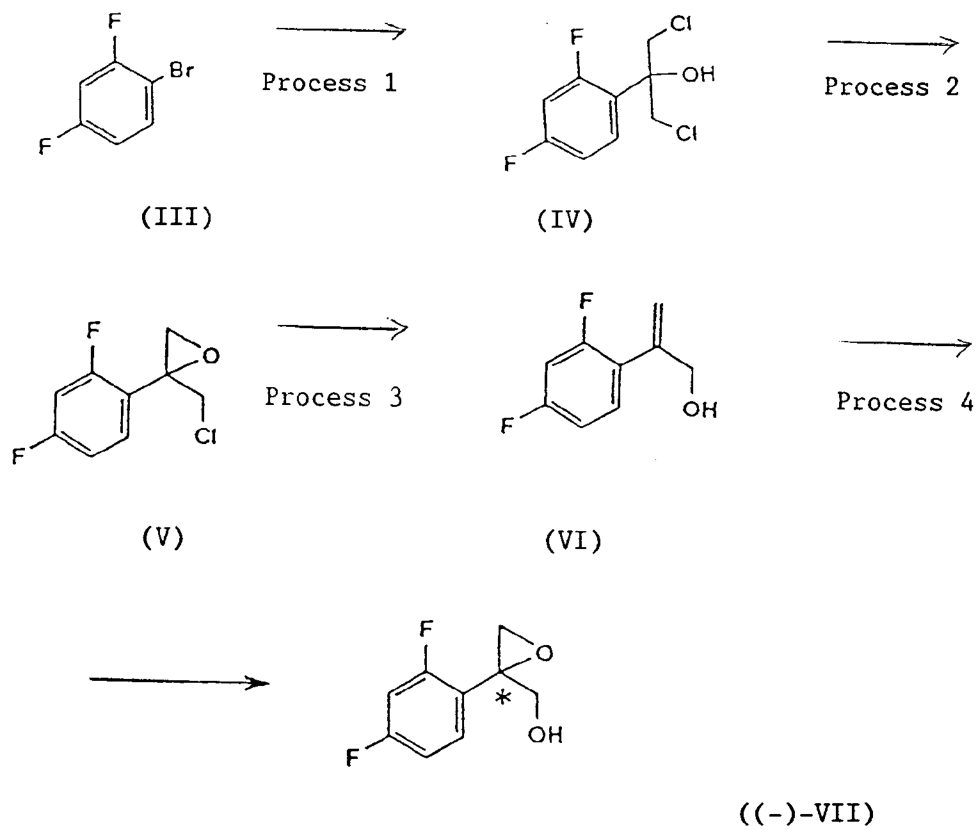


(III) (IV) (V) (VI) ((-)-VII)

Process 1 is the condensation of 1,3-dichloroacetone with 2,4-difluorobromobenzene (III) and can be performed by a well-known literature method (for example JPS 58-32868) for example by reacting them in the presence of a base such as n-butyl lithium in an organic solvent such as anhydrous ether, hexane etc. Process 2 is a process of ring closure to the epoxide. It can for example be performed by reaction in the presence of bases such as sodium hydride, in organic solvents such as dimethylformamide. Process 3 is an allyl alcohol synthesis process. This process can be performed by generation of telluride ion in the reaction system by reacting a reducing agent such as sodium hydroxymethanesulphinate, sodium borohydride or lithium triethylborohydride with tellurium in the presence of a base such as sodium hydroxide, then reacting this with 1-chloro-2-(2,4-difluorophenyl)-2,3-epoxypropane V. Also, in this process it is possible to use selenium instead of tellurium. Furthermore, this process can also be performed by reducing the compound V with for example zinc in an organic solvent such as dimethylformamide. Process 4 is an asymmetric oxidation process. By oxidation of 2-(2,4-difluorophenyl)allyl alcohol VI with t-butyl hydroperoxide in organic solvents such as methylene chloride in the presence of a titanium tetraalkoxide such as titanium tetraisopropoxide or titanium tetrabutoxide and of a dialkyl (+)-tartrate such as diethyl (+)-tartrate, diisopropyl (+)-tartrate or dicyclohexyl (+)-tartrate, (-)-2-(2,4-difluorophenyl)-2,3-epoxypropanol ((-)-VII) can be obtained in high optical purity. Further, if in this process instead of dialkyl (+)tartrate a dialkyl (-)-tartrate is used, (+)-2-(2,4-difluorophenyl)-2,3-epoxypropanol ((+)-VII) is obtained in high yield.

Further, compound VI can also be prepared as follows. Namely, by reacting compound IV with a Grignard reagent such as ethylmagnesium bromide in an organic solvent such as ether in the presence of ferric chloride etc, 1-(2,4-difluorophenyl)-cyclopropanol is obtained, and then by converting the hydroxyl group into a sulphonate ester using methanesulphonyl chloride or p-toluenesulphonyl chloride in the presence of a base such as triethylamine, and treating this with a base such as calcium carbonate, compound VI can be obtained. Moreover, it is also possible to prepare compound VI as follows: after sythesizing 2-(2,4-difluorophenyl)-1,2-epoxypropane by reacting 2,4-difluorobenzophenone with a methylating agent such as trimethylsulphoxonium iodide in the presence of a base such as sodium hydride, compound VI can be obtained by reacting this with trimethylsilyl iodide in the presence of a base such as 1,8-diazabicyclo[5.4.0]undec-7-ene.

The optically active compounds VII can further be prepared as follows:

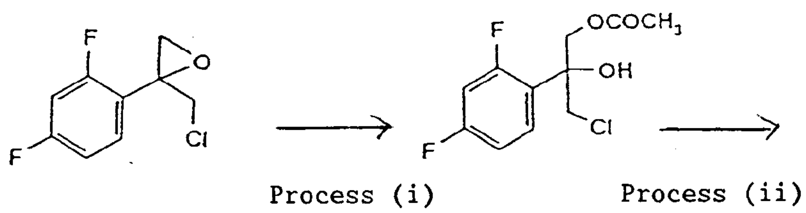


(V) (VIII)

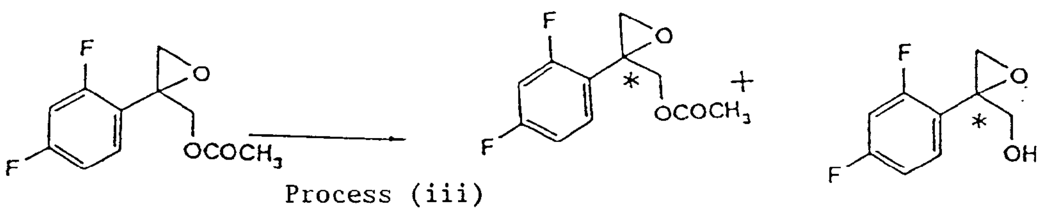


(IX) ((-)-IX) ((+)-VII)

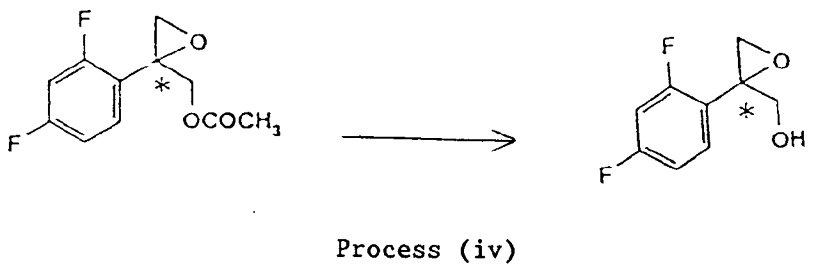


((-)-IX) ((-)-VII)

Process (i) is an epoxide ring opening reaction, and can be perfomed by reacting compound V with sodium acetate in an organic solvent such as acetic acid. Process (ii) is an epoxide recyclisation process. It can for example be performed by reaction in the presence of a base such as sodium hydride in an organic solvent such as dimethoxyethane. Process (iii) is an enzymatic asymmetric ester hydrolysis process, and can be performed using a hydrolytic enzyme such as an esterase or a lipase in a buffer solution or in a mixture solvent of buffer solution and an organic solvent such as diisopropyl ether, ethanol, acetone or dimethylformamide. In this process, the (+)-form undergoes ester hydrolysis selectively, and (+)-2-(2,4-difluorophenyl)-2,3-epoxypropanol ((+)-VII) is obtained. Meanwhile, the (-)-1-acetoxy-2-(2,4-difluorophenyl-2,3-epoxypropane ((-)-IX) does not undergo ester hydrolysis and is recovered from the reaction liquid in high optical purity. Process (iv) is a normal ester hydrolysis, and by hydrolysis of the compound of formula ((-)-IX recovered in process (iii), using bases such as potassium hydroxide or sodium hydroxide, (-)2-(2,4-difluorophenyl)-2,3-epoxypropanol ((-)-VII) is obtained.

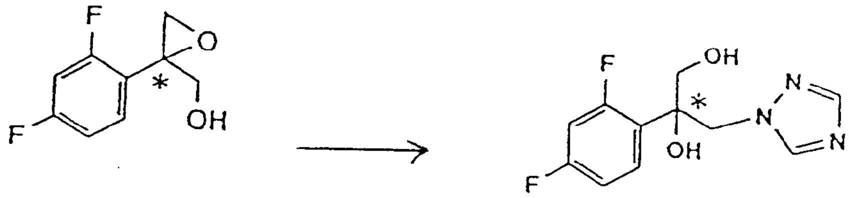

(-)-VII Process 5 ((-)-X)

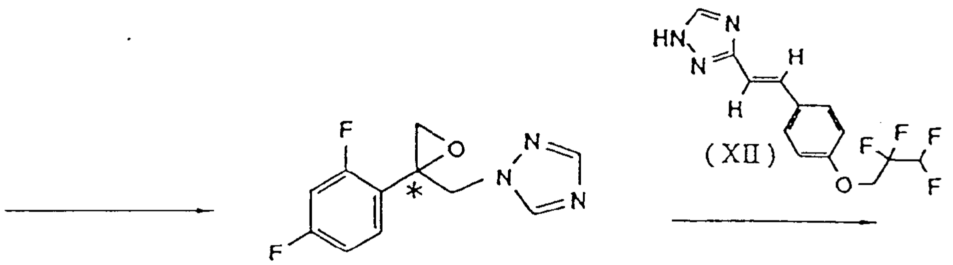

(XII)

Process 6 ((+)-XI) Process 7

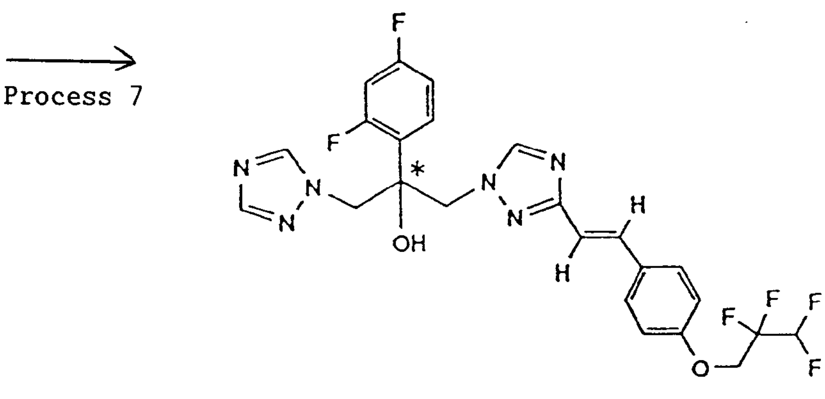

Process 7

((+)-I)

Process 5 is a process of triazole condensation. By reacting compound ((-)-VII) with 1,2,4-triazole in an organic solvent such a tetrahydrofuran or dimethylformamide in presence of a base such as potassium carbonate, potassium hydrogen carbonate or sodium hydride, (-)-2-(2,4-difluorophenyl)-3-(1H-1,2,4-triazol-1-yl)propan-1,2-diol ((-)-X can be obtained. Further, by recrystallisation of this compound from organic solvents such as chloroform, it can be obtained as the optically pure substance. Process 6 is an epoxide ring closure process. Firstly, the primary hydroxy group is converted to a sulfonate ester by reaction with methanesulfonyl chloride or p-toluenesulfonyl chloride in an organic solvent such as ether or tetrahydrofuran in the presence of an excess of a base such as triethylamine or pyridine, then if this reaction mixture is treated with water the epoxidation

takes place through the action of the excess base present in the system, and (+)-2-(2,4-difluorophenyl)-3-(1H-1,2,4-triazol-1-yl)-2,3-epoxypropane ((+)-XI) can be obtained. In this process it is also possible to isolate the sulfonate ester, but for ease of operation the method omitting its isolation is preferable. Process 7 is a process of condensation with the substituted triazole. By reaction of compound ((+)-XI) in an organic solvent such as tetrahydrofuran, dimethylformamide or ethanol, in presence of a base such as potassium carbonate, potassium bicarbonate or sodium hydride, with the compound 3-[(E)-4-(2,2,3,3-tetrafluoropropoxy)styryl]-1H-1,2,4-triazole (XII) well-known from the original literature (see for example JPS Sho 61-72767), (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)pro pan-2-ol ((+)-I) can be obtained. Further, by recrystallisation of this compound from organic solvents such as ethyl acetate, ether, hexane or solvent mixtures thereof, it can be obtained as the optically pure substance.

In this preparation method, it is also possible to obtain the compound of the formula (+)-I using the compound (+)-VII as the starting material instead of the compound (-)-VII, by changing the order of introduction of the 1,2,4-triazole and the substituted triazole (XII). That is to say, compound (+)-I can also be prepared as follows:

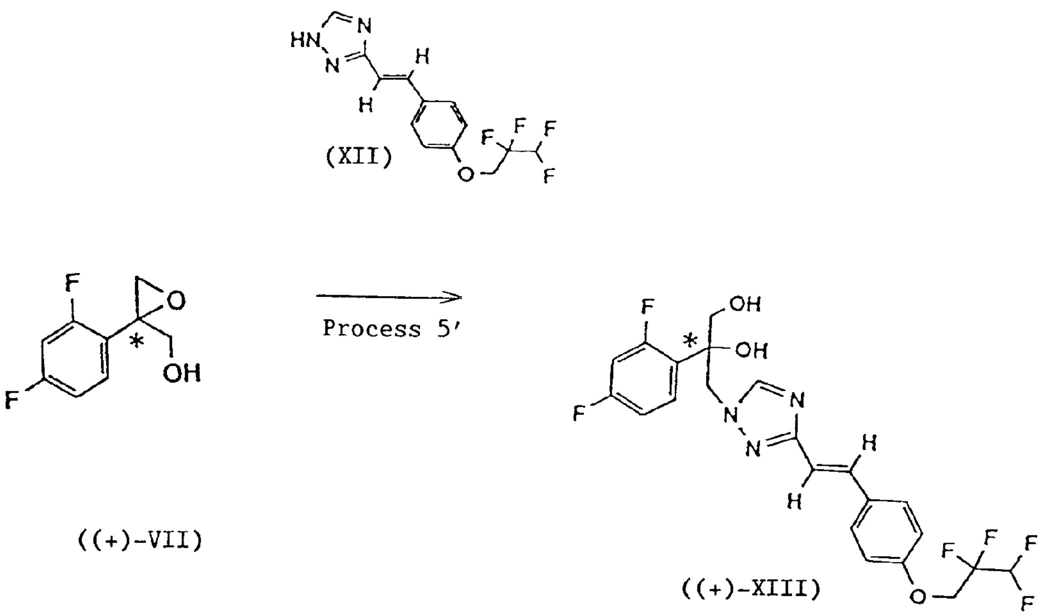


(XII)

((+)-VII)

((+)-XIII)

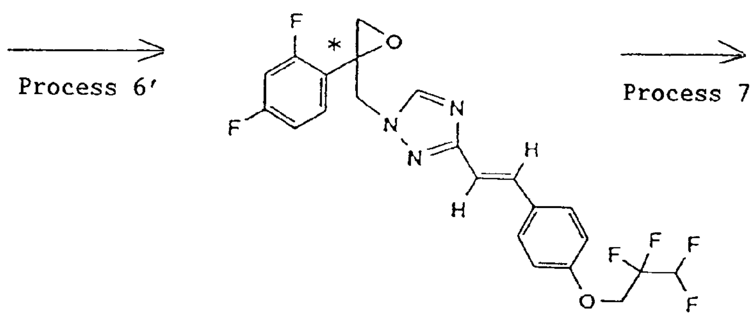


((-)-XIV)

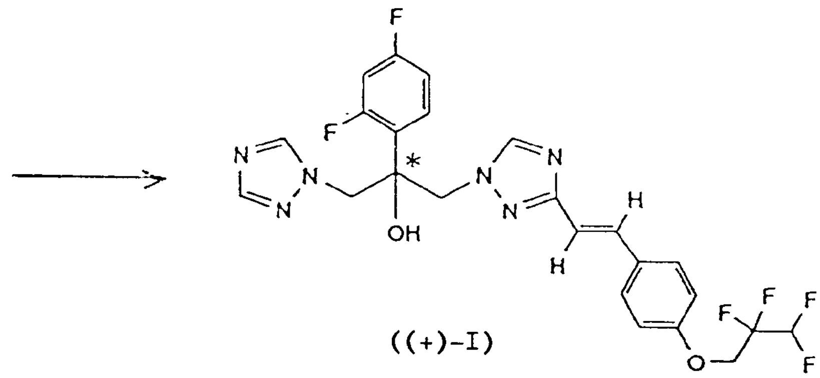

((+)-I)

Process 5′ is a process of condensation with the substituted triazole. By reacting compound ((+)-VII) with 3-[(E)-4-(2,2,3,3-tetrafluoropropoxy)styryl]-1H-1,2,4-triazole in an organic solvent such as tetrahydro furan or dimethylformamide in the presence of a base such as potassium carbonate, potassium hydrogen carbonate or sodium hydride, the (+)-2-(2,4-difluorophenyl)-3-(1H-1,2,4-triazol-1-yl)propan-1,2-diol ((+)-XIII) can be obtained. Process 6′ is an epoxide ring closure process. Firstly, the primary hydroxy group is converted to a sulfonate ester by reaction with methanesulfonyl chloride or p-toluenesulfonyl chloride in an organic solvent such as ether, tetrahydrofuran or dimethylformamide in presence of a base such as triethylamine or pyridine, then by converting the sulfonate ester obtained to the epoxide using a base such as sodium hydride, (-)-2-(2,4-difluorophenyl)-3-(1H-1,2,4-triazol-1-yl)-2,3-epoxypropane ((-)-XIV) can be obtained. In this process it is also possible to isolate the sulfonate ester. Process 7′ is a process of condensation with triazole. By reaction of compound ((-)-XIV) with 1,2,4-triazole in an organic solvent such as tetrahydrofuran or dimethylformamide in the presence of a base such as potassium carbonate, potassium bicarbonate or sodium hydride, (+)-2-(2,4-difluorophenyl)-1-[3-[(E)-4-(2,2,3,3-tetrafluoropropoxy)-styryl]-1H-1,2,4-triazol-1-yl]-3-(1H-1,2,4-triazol-1-yl)propan-2-ol ((+)-I) can be obtained.

Also, the compound XII used in the present method of preparation is known from the original literature (see for example JPS Sho 61-72767), but since in the previous method of preparation there are various problems, such as that the total yield from the starting material 4-chlorobenzonitrile (XV) is low, the cis-trans isomer selectivity at the double bond is poor, and also it involves many processes in which purification by silica gel column chromatography is necessary, it is extremely difficult to operate it industrially. In the present invention, the aforesaid problems have been solved and an industrially practicable route to compound XII has been discovered. Namely, compound XII can be prepared as follows.

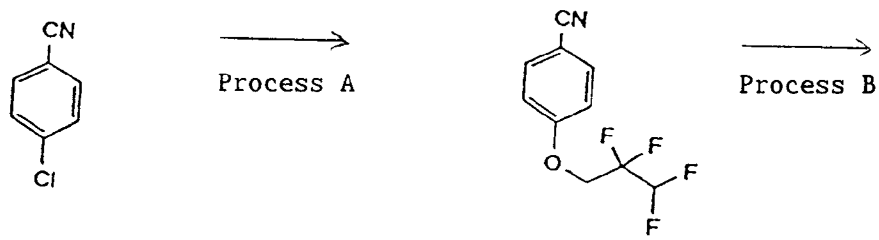


(XV) (XVI)

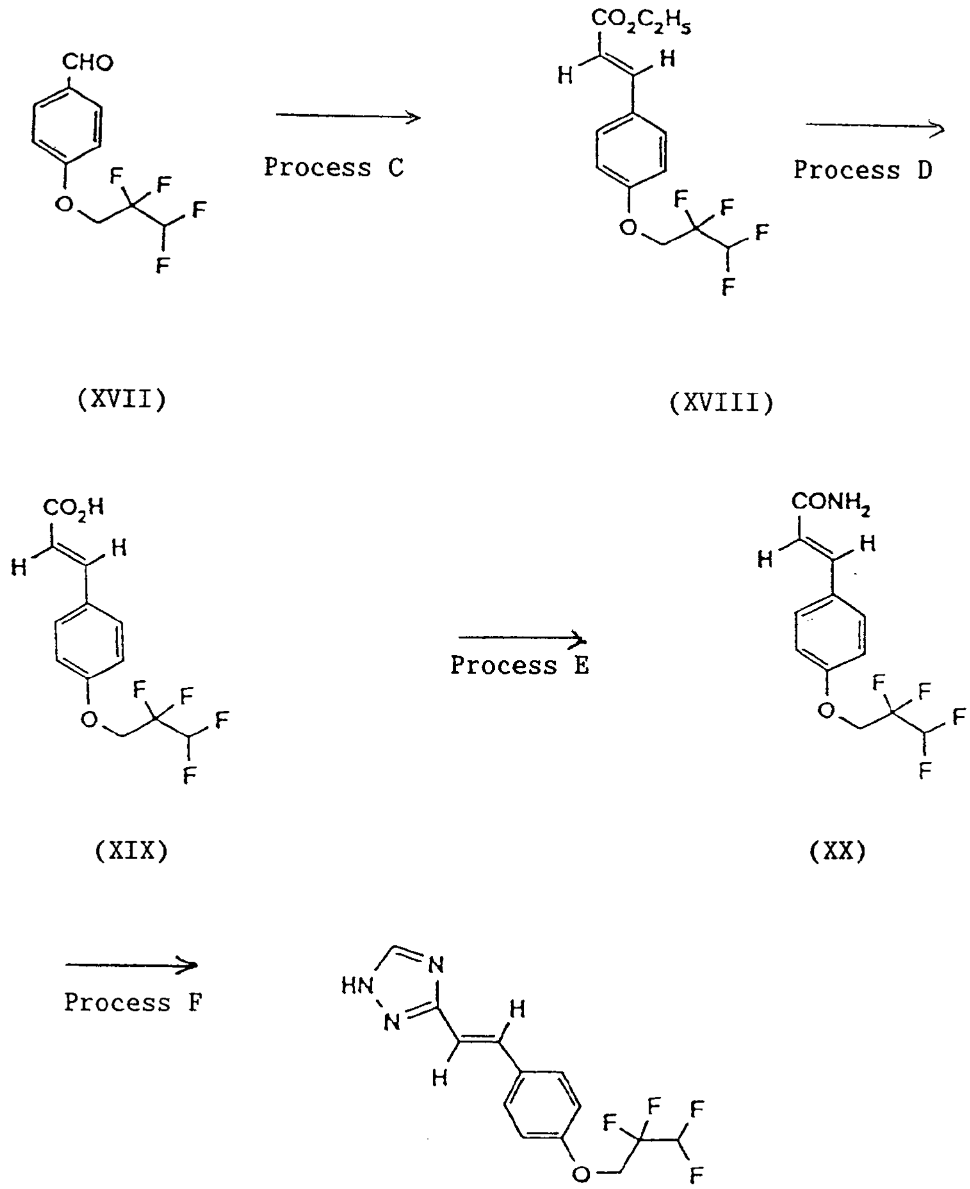


(XVII) (XVIII) (XIX) (XX)

(XII)

Process A is a known literature method (J P Idoux et al., J. Org. Chem. 48, 3772, 1983). This process can be performed by reacting 4-chlorobenzonitrile (XV) and 2,2,3,3-tetrafluoropropanol in the presence of a base such as sodium hydride in an organic solvent such as dimethylformamide or dimethylsulfoxide. Moreover, the compound XVI obtained in this process can be purified by recrystallisation from a mixture of an organic solvent such as isopropanol and water. Process B is a process of reduction of the cyano group to a formyl group and is a known literature method (JPS Sho 61-72767). This process can be performed by using a reducing agent such as diisobutylaluminium hydride in toluene. Process C is a process of introducing a double bond. In this process, ethyl (E)-4-(2,2,3,3-tetrafluoropropoxy)-cinnamate (XVIII) is obtained by reacting compound XVII and ethyl diethylphosphonoacetate in an organic solvent such as dimethoxyethane or tetrahydrofuran in the presence of a base such as potassium hydroxide or sodium hydride. Moreover, this reaction proceeds stereoselectively, only the desired (E)-form being produced, and because production of the (Z)-form is not observed the need for purification e.g. by silica gel column chromatography is eliminated. Process D is a normal ester hydrolysis. It can be performed using a base such as sodium hydroxide or potassium hydroxide in water or an organic solvent such as ethanol or dioxan or a mixture thereof. Process E is a normal amidation. In this process, after conversion of compound XIX to an acid halide with a halogenating reagent such as thionyl chloride or phosphorus pentachloride, compound XX can be obtained by addition of concentrated aqueous ammonia. Moreover, in this process it is also possible to isolate and purify the acid halide, but for ease of operation it is

preferable to obtain compound XX without isolating and purifying this. Process F is the process of constructing the triazole ring. In this process, after firstly converting to the imino ether using an alkylating agent such as trimethyloxonium tetrafluoroborate in an organic solvent such as methylene chloride, next, by reaction with formyl hydrazide in an organic solvent such as ether, 3-[(E)-4-(2,2,3,3-tetrafluoropropoxy)-styryl]-1H-1,2,4-triazole (XII) can be obtained. Further, in this process, it is also possible to isolate and purify the imino ether, but for ease of operation it is preferable to obtain compound XII without isolating and purifying this.

Further, from the results of the X-ray crystal analysis described below in the practical examples, compound (-)-X was confirmed to give the (R) absolute configuration. Since the reactions process 5, process 6, process 7, process (iv), process 5′, process 6′ and process 7′ are all stereoretentive reactions, among the compounds mentioned in the present invention the absolute configurations of the compounds stated below are as shown below.

| Compound | Absolute Configuration |
|---|---|
| (+)-I | (R) |
| (-)-I | (S) |
| (-)-VII | (S) |
| (+)-VII | (R) |
| (-)-IX | (R) |
| (-)-X | (R) |
| (+)-XI | (R) |
| (+)-XIII | (S) |
| (-)-XIV | (S) |

Below, the effectiveness, mode of use, dosage and method of preparation of medicines are described for the active ingredient of the present invention, (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol (compound (+)-I) and its salts.

Firstly, in order to show the effectiveness of the compound of the invention, we present data on its antifungal activity.

## Test Compounds

Compound (+)-I: (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol.

Compound (-)-I: (-)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol.

Compound (±)-I (compound A): (±)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol.

Fluconazole: 2-(2,4-difluorophenyl)-1,3-bis(1H-1,2,4-triazol-1-yl)-propan-2-ol.

## Test Example 1

### Measurement of Minimum Inhibitory Concentration (MIC)

Yeast-type fungi were cultured in Sabouraud dextrose broth (SDB, Difco) at 30°C for 24 hours, and $10^5$ cell/ml suspension for inoculation were prepared by diluting this with sterile physiological saline. In the case of filamentous fungi, 3 to 5 ml sterile physiological saline containing 0.05% (w/v) Tween 80 were added to mature Sabouraud dextrose agar culture medium (SDA, Difco) slant cultures (cultured for 2 to 3 weeks) in which conidia had formed abundantly, and the conidia were separated using an inoculating loop (Nunc Inc.). The conidial suspensions obtained were collected using a sterile pipette, and, after filtration through a double layer of sterile gauze to remove mycelial aggregates and agar fragments, were made into suspensions for inoculation by dilution of $10^5$ conidia/ml with sterile physiological saline. Meanwhile, as regards the drug-containing agar plates, a drug concentration of 0.005 to 10 mg/ml was prepared using dimethyl sulfoxide (DMSO, Wako Junyaku), and, by adding 99 parts of SDA to 1 part of this drug solution, two-fold serial dilution agar media of final dilutions

0.05 to 100 µg/ml were prepared. The fungal suspensions for inoculation prepared by the above method were inoculated onto these agar plates using a MIT-P microplanter (Sakuma Seisakusho). The agar plates were static-cultured at 30°C for 48 hours, and the lowest concentration at which no fungal growth at all was observed with the naked eye was taken to be the minimum inhibitory concentration (MIC).
The results are shown in table 1.

Table 1 - MIC (µg/ml)

| | COMP. (+)I | COMP. (-)I | COMP. (±)I | FLUCONAZOLE |
|---|---|---|---|---|
| Candida albicans IFM 40009 | 12.5 | 12.5 | 12.5 | >100 |
| Candida tropicalis TIMM 0313 | 12.5 | >100 | 12.5 | >100 |
| Candida glabrata TIMM 1064 | 0.78 | >100 | 1.56 | 100 |
| Cryptococcus neoformans TIMM 0362 | 0.39 | >100 | 0.78 | 50 |
| Trichophyton mentagrophytes TIMM 1188 | 0.10 | 50 | 0.39 | 25 |
| Aspergillus fumigatus IFM 4942 | 6.25 | >100 | 12.5 | >100 |

**Test Example 2**

**Measurement of Effectiveness in Treatment of In vivo Infections**

Using 10 animals per group, male ICR mice (body weight 18 to 20 g) were infected by intravenous injection of the test fungi as an aqueous suspension. The test compounds were given orally once 1 hour after infection, or once daily for 5 days starting from 1 hour after infection. After 2 weeks, the number of animals surviving was counted, and the dosage at which 50% of the animals survive ($ED_{50}$: mg/kg) was calculated using the Litchfield-Wilcoxon method.
The results are shown in table 2.

Table 2 - $ED_{50}$ (mg/kg)

| | NO. OF TIMES DOSED | COMP. (+)I | COMP. (±)I | FLUCONAZOLE |
|---|---|---|---|---|
| Candida albicans IFM 40009 | 1x | 2.6 | NOT MEASURED | >30 |
| Candida albicans IFM 40009 | 5x | 1.0 | 1.7 | 3.6 |
| Cryptococcus neoformans TIMM 0362 | 1x | 19.7 | 43.5 | >100 |
| Aspergillus fumigatus IFM 4942 | 5x | 39.8 | 53.6 | 57.9 |

**Test Example 3**

**(1) Toxicity Testing of Compound A ((±)-I in Monkeys**

Compound A was given orally to monkeys every day for 28 days at dosages of 1, 3, 10 and 30 mg/kg/day, and its toxicity was studied.

As a result, at dosages of 3 mg/kg/day or more, a decrease in serum cortisol, hyperplasia and increase in lipid droplets in the zona fasciculata in the adrenal cortex, and atrophy of the seminiferous tubules of the testes were observed; at the dosage of 30 mg/kg/day, prolongation of the QTc interval in the electrocardiogram (QT period corrected for heart rate) was also observed. Under these test conditions, it was considered that 1 mg/kg/day was a safe dosage.

Also, the results of a study of the variation in the plasma concentrations of compound A using these animals showed that the concentrations in the plasma increased day-by day, and reached a steady state after 2 to 3 weeks.

**(2) Toxicity Testing of ((+)-I) in Monkeys**

Compound (+)-I was given orally to monkeys every day for 28 days at dosages of 1, 3, 10 and 30 mg/kg/day, and its toxicity studied.

As a result, at dosages of 10 mg/kg/day or more, slight hyperplasia and increase in lipid droplets in the zona fasciculata in the adrenal cortex was observed, but there was no decrease in the serum cortisol level. Apart from these slight histological changes, no abnormalities in the electrocardiogram were seen, and no toxicity whatever was observed. Under these test conditions, it was considered that 30 mg/kg/day was safe dosage.

Also, the results of a study of the variation in the plasma concentrations of compound (+)-I using these animals showed that there was no day-by day increase in the plasma concentrations.

As is clear from the above test results, it was confirmed that compound (+)-I of this invention, the salts, solvates and the solvates of the salts thereof show an anti-fungal activity strikingly stronger than fluconazole, both in vivo and in vitro. On the other hand, compared with compound (+)-I of this invention, its enantiomer, compound (-)-I, showed strikingly weaker antifungal activity. Further, in compound (+)-I of this invention, its pharmacological activity has clearly been separated from the side-effects observed with compound A. Because

of the above, medicaments made with compound (+)-I of this invention as the active ingredient have a high degree of safety, and can be offered as exceptionally valuable therapeutic agents against fungal infections

The compound (+)-I of this invention can be made into a variety of drug forms by appropriate selective combination of excipients, binders, lubricants, colourants, flavourings, suspension agents or emulsifiers (for example polysorbate 80, gum arabic, etc.) appropriate types of generally used carriers or solvents, for example sterile water as needed or vegetable oil, and also pharmaceutically acceptable solvents or solution aids (for example alcohol, glycerine, propylene glycol), etc.

As such drug forms, tablets, capsules, granules, microgranules, powders, suppositories, syrups, inhalation drugs, soft ointments, emulsions, suspensions, liquids for eye-drops, aqueous or non-aqueous injectables, emulsion or suspension injectables, or solid injectables used by dissolving, emulsifying or suspending at the time of use, may be mentioned, and may be adminstered to the patient either orally or parenterally (for example intravenously, intramuscularly, subcutaneously, intrarectally, by percutaneous absorption or transmucosal absorption), or by application in a pessary, etc. In the case of tablets, capsules, powders, injectables, suppositories (systemic) or other systemic administrations, for the daily dosage, calculated in terms of the amount of the compound of the invention, administration of 0.1 mg - 2000 mg, preferably 1 mg - 200 mg, is desirable, and this can be appropriately varied depending on the condition of the patient. It is also possible to give the whole amount at a single time or to divide it between 2-6 times or to give it as an intravenous drip, etc.

The compound of the present invention, as well as possessing powerful antifungal activity, has a high degree of safety as a result of the clearcut separation of its pharmacological activity from the toxicity of compound A. Consequently medicaments with the compound of this invention as active ingredient are extremely valuable as drugs for the treatment of fungal infections or prophylactic drugs. Also this invention provides a valuable method for preparing this outstanding optically active antifungal agent industrially and at the same time stereoselectively.

Below, the invention is illustrated in more detail by means of practical examples. Furthermore, this invention is not limited by the examples below. Also, the optical purities mentioned in the practical examples were determined by HPLC measurement (eluent ethanol:diethylamine = 100:1 using a column containing an optical active support (Daicel Chiralcel OD).

## Example 1

### Synthesis of 4-(2,2,3,3-tetrafluoropropoxy)benzonitrile

14.4 g sodium hydride (60% oil) were suspended in 135 ml dimethylformamide previously dried for 1 day by addition of 13.5 g molecular sieve 4A, and 43.2 g 2,2,3,3-tetrafluoropropanol were added dropwise with ice-cooling. After the foaming had subsided, 30 g 4-chlorobenzonitrile were added dropwise with ice-cooling, and the mixture was stirred for 5 hours at 40°C. After addition of 600 ml 5% hydrochloric acid solution to the reaction mixture, the crystals deposited were collected by filtration, and recrystallisation from a 1:4 water:isopropanol mixture (330 ml) gave 33.7 g of the title compound.

M.Pt. 79.9-80.2°C

IR spectrum: (KBr pellet) $\nu$ $cm^{-1}$:

2224, 1606, 1509, 1264, 1100.

NMR spectrum: (90 MHz, $CDCl_3$) $\delta$ ppm:

7.65 (2H, d, J = 8.9 Hz); 7.00 (2H, d, J = 8.9 Hz); 6.03 (1H, tt, J = 53.1, 4.3 Hz); 4.41 (2H, tt, J = 11.9, 1.7 Hz).

## Example 2

### Synthesis of 4-(2,2,3,3-tetrafluoropropoxy)benzaldehyde

167 g of the 4-(2,2,3,3-tetrafluoropropoxy)benzonitrile obtained in example 1 were dissolved in 680 ml toluene and 621 ml diisobutylaluminium hydride (1.5 M toluene solution) were added dropwise with ice-cooling. After stirred at room temperature for 2 hours, 350 ml toluene were added to the reaction mixture, and 280 ml methanol were added dropwise. Next, 1000 ml 2M hydrochloric acid were added to the reaction mixture, and after stirring for 1 hour the liquids were separated and the aqueous layer extracted with ethyl acetate. The combined organic layers were concentrated under reduced pressure, 800 ml methanol and 450 ml 1M hydrochloric acid were added to the residue, and the mixture stirred with heating at 40°C for 3 hours. The methanol was distilled off under reduced pressure, 500 ml 1M hydrochloric acid were added to the residue, and the mixture was extracted with ethyl acetate. After washing with saturated sodium chloride solution, and drying with anhydrous sodium sulphate, the solvent was distilled off under reduced pressure to yield 155.4 g of the title compound as an oil.

IR spectrum (neat) ν $cm^{-1}$:
1762, 1701, 1604, 1585, 1264, 1106.
NMR spectrum: (90 MHz, $CDCl_3$) δ ppm:
9.90 (1H, s), 7.87 (2H, d, J = 8.6 Hz); 7.06 (2H, d, J = 8.6 Hz); 6.11 (1H, tt, J = 53.1, 4.6 Hz); 4.45 (2H, t, J = 11.9 Hz).

## Example 3

### Synthesis of ethyl (E)-4-(2,2,3,3-tetrafluoropropoxy)cinnamate

11.6 g powdered potassium hydroxide were suspended in 640 ml tetrahydrofuran, 23.3 g ethyl diethylphosphonoacetate were added dropwise to this, and then 24.5 g of the 4-(2,2,3,3-tetrafluoropropoxy)benzaldehyde obtained in example 2 were added dropwise. After completion of the addition, the reaction mixture was poured into water, and the crystals deposited were collected by filtration, to yield, after air-drying, 25.7 g of the title compound.
M.Pt 41.1-42.6°C
IR spectrum: (KBr pellet) ν $cm^{-1}$:
1701, 1637, 1605, 1515, 1180, 1121, 834.
NMR spectrum (90 MHz, $CDCl_3$) δ ppm:
7.64 (1H, d J = 16.2 Hz); 7.50 (2H, d, J = 8.9 Hz); 6.93 (2H, d, J= 8.9 Hz); 6.33 (1H, d, J= 16.2 Hz); 6.05 (1H, tt, J = 53.1, 4.8 Hz);
4.37 (2H, t, J = 11.9 Hz); 4.26 (2H, q, J = 7.1 Hz); 1.33 (3H, t, J = 7.1 Hz).

## Example 4

### Synthesis of (E)-4-(2,2,3,3-tetrafluoropropoxy)cinnamic acid

25 g of the ethyl (E)-4-(2,2,3,3-tetrafluoropropoxy)-cinnamate obtained in example 3 were dissolved in 250 ml ethanol, and a solution of 7.5 g sodium hydroxide in 50 ml water was added dropwise at room temperature. After 1 hour, the crystals which had been deposited were dissolved by addition of 50 ml water, then the ethanol was distilled off under reduced pressure, 1200 ml water were added to the residue, and adjusted to pH 1 with concentrated hydrochloric acid. The mixture was extracted with 1000 ml ethyl acetate, and after washing the ethyl acetate layer with satured sodium chloride solution, this was dried with anhydrous sodium sulphate. After concentration under reduced pressure, the residue was washed with hexane, the crystals obtained were filtered off and air-dried to yield 20.5 g of the title compound.
M.Pt. 177.4-178.7°C
IR spectrum: (KBr pellet) ν $cm^{-1}$:
1676, 1627, 1603, 1512, 1220, 1176, 1121, 1112, 832.
NMR spectrum: (90 MHz, DMSO-$d_6$) δ ppm:
12.2 (1H, bs); 7.67 (2H, d, J = 8.6 Hz); 7.55 (1H, d, J = 15.8 Hz);
7.07 (2H, d, J= 8.6 Hz); 6.66 (1H, tt, J = 52.0, 5.6 Hz); 6.40 (1H, d, J = 15.8 Hz); 4.63 (2H, t, J = 13.5 Hz).

## Example 5

### Synthesis of (E)-4-(2,2,3,3-tetrafluoropropoxy)cinnamamide

20 g of the (E)-4-(2,2,3,3-tetrafluoropropoxy)cinnamic acid obtained in example 4 were heated under reflux for 1 hour with 50 ml thionyl chloride. After completion of the reaction, the mixture was concentrated under reduced pressure, the residue was dissolved in 135 ml toluene, and this was added dropwise to 50 ml concentrated aqueous ammonia. The reaction mixture which had depostited crystals was extracted by addition of 200 ml ethyl acetate, and this was successively washed with water and saturated sodium chloride solution, and dried over anhydrous sodium sulphate. After concentration of the ethyl acetate layer under reduced pressure, the residue was washed with 100 ml of 6:1 hexane:ether mixed solvent, and the crystals deposited were collected by filtration to yield 17.5 g of the title compound.
M.Pt 147.9-149.8°C
IR spectrum: (KBr pellet) ν $cm^{-1}$:
3182, 3172, 1670, 1598, 1515, 1399, 1259.
NMR spectrum: (90 MHz, DMSO-$d_6$) δ ppm:
7.53 (2H, d, J = 8.8 Hz); 7.37 (1H, d, J = 15.8 Hz); 7.07 (2H, d, J = 8.8 Hz); 6.65 (1H,tt, J = 51.9, 5.1 Hz); 6.47 (1H, d J = 15.8 Hz);
4.60 (2H, t, J = 13.6 Hz).

## Example 6

**Synthesis of 3-[(E)-4-(2,2,3,3-tetrafluoropropoxy)styryl]-1H-1,2,4-triazole**

52.9 g of the (E)-4-(2,2,3,3-tetrafluoropropoxy)cinnamamide obtained in example 5 were dissolved in 920 ml anhydrous methylene chloride, and 42.4 g trimethyloxonium tetrafluoroborate were added. After refluxing for 5 hours, the reaction mixture was poured into 1000 ml saturated ice-water solution of sodium carbonate, and the methylene chloride layer was separated. The organic layer was washed with satured aqueous sodium chloride solution and dried over anhydrous sodium sulphate. The solvent was distilled off under reduced pressure, 265 ml anhydrous ethanol were added to the residue, and after addition of 17.2 g formyl hydrazide and 13.3 ml triethylamine the mixture was heated under reflux for 2 hours. The reaction mixture was concentrated, the residue was dissolved in 600 ml ethyl acetate, washed successively with water and satured sodium chloride solution, and dried over anhydrous sodium sulphate. The solvent was distilled off under reduced pressure, and the residue was washed by adding ether, to yield 36.6 g of the title compound.

M.Pt 153.2-154.6°C

IR spectrum: (KBr pellet) $\nu$ $cm^{-1}$:

1607, 1515, 1258, 1110.

NMR spectrum: (90 MHz, DMSO-$d_6$) $\delta$ ppm:

8.26 (1H, s); 7.63 (2H, d, J = 8.8 Hz); 7.48 (1H, d, J = 17.2 Hz);

7.07 (2H, d, J = 8.8 Hz); 7.03 (1H, d, J = 17.2 Hz); 6.68 (1H, tt, J = 51.9, 5.7 Hz); 4.62 (2H, t, J =13.6 Hz).

## Example 7

**Synthesis of 1,3-(dichloro)-2-(2,4-difluorophenyl)propan-2-ol**

225 g of 2,4-difluorobromobenzene were dissolved in 2000 ml anhydrous ether, and 733 ml n-butyl lithium (1.6 M hexane solution) were added dropwise at -60°C. After 1 hour, 148 g 1,3-dichloroacetone were dissolved in 300 ml anhydrous ether and added dropwise at -50°C. After stirring for 30 minutes, a solution of 76 g acetic acid in 200 ml anhydrous ether was added dropwise. After addition of 500 ml water with ice-cooling, the liquids were separated and the ether layer was dried with anhydrous sodium sulphate and the solvent distilled off under reduced pressure to yield 279 g of the title compound as an oil.

IR specrum: (neat) $\nu$ $cm^{-1}$

3516, 1618, 1599, 1501, 1424, 1267.

NMR specrum: (90 MHz, $CDCl_3$) $\delta$ ppm:

7.83-7.55 (1H, m); 7.05-6.70 (2H, m); 4.03(4H, s); 3.02 (1H, bs).

## Example 8

**Synthesis of 1-chloro-2-(2,4-difluorophenyl)-2,3-epoxypropane**

45 g sodium hydride (60% oil) were suspended in 400 ml dimethylformamide, and a solution of 242 g of the 1,3-dichloro-2-(2,4-difluorophenyl)propan-2-ol obtained in example 7 in 100 ml dimethylformamide was added dropwise with ice-cooling. The reaction mixture was poured into 750 ml ice-water, and adjusted to pH 4 with concentrated hydrochloric acid. The mixture was extracted by adding 1500 ml ethyl acetate, the ethyl acetate layer was washed successively with aqueous saturated sodium hydrogen carbonate solution and saturated sodium chloride solution, and dried over sodium sulphate. The solvent was distilled off under reduced pressure, and the residue was distilled under reduced pressure, the fraction distilling at 125-135°C/30 mm Hg being collected to yield 99.5 g of the title compound.

B.Pt. 125-135°C/30 mm Hg.

IR spectrum (neat) $\nu$ $cm^{-1}$:

1619, 1602, 1508, 1425, 1272.

NMR spectrum: (90 MHz, $CDCl_3$) $\delta$ ppm:

7.57-7.31 (1H, m); 6.98-6.71 (2H, m); 4.09 (1H, d, J = 11.9 Hz); 3.68 (1H, d, J = 11.9 Hz); 3.20 (1H, d, J = 4.8 Hz); 2.93 (1H, d, J = 4.8 Hz).

## Example 9

**Synthesis of 2-(2,4-difluorophenyl)allyl alcohol**

60.3 g metallic tellurium and 109.7 g sodium hydroxymethane-sulphinate were suspended in 2500 ml 1M aqueous sodium hyroxide solution, and the mixture was stirred under a nitrogen atmosphere for 1 hour at 55-60°C. To this solution, a solution of 94 g of the 1-chloro-2-(2,4-difluorophenyl)-2,3-epoxypropane obtained in

example 8 in 500 ml dioxan was added dropwise over 30 minutes. After stirring for 30 minutes, the reaction mixture was cooled to room temperature, insoluble material was removed by filtration, air was passed into the filtrate for 30 minutes, and the material deposited was again filtered. The filtrate was extracted by addition of 1000 ml ether, and the ether layer was washed with saturated sodium chloride solution and dried over anhydrous sodium sulphate. The solvent was distilled off under reduced pressure to yield 77 g of the title compound as an oil.
IR spectrum: (neat) $\nu$ $cm^{-1}$:
3340, 1617, 1506, 1420, 1267.
NMR spectrum: (90 MHz, $CDCl_3$) $\delta$ ppm:
7.44-7.18 (1H, m); 6.96-6.69 (2H, m); 5.51 (1H, m); 5.35 (1H, bs), 4.46 (2H, m); 1.71-1.64 (1H, m).

**Example 10**

**Synthesis of (-)-2-(2,4-difluorophenyl)-2,3-epoxypropanol**

197 ml titanium tetraisopropoxide were dissolved in 1700 ml methylene chloride, cooled to -20°C under a nitrogen atmosphere, and a solution of 164 g diethyl (+)-tartrate in 100 ml methylene chloride was added. After stirring for 15 minutes, a solution of 113 g of the 2-(2,4-difluorophenyl)allyl alcohol obtained in example 9 in 100 ml methylene chloride was added dropwise. After stirring for 15 minutes, 332 ml of a 7.1M methylene chloride solution of t-butyl hydroperoxide was added dropwise, and the mixture stirred for 8 hours at -20°C. After gradual addition of 1000 ml 10% aqueous tartaric acid soluton to the reaction mixture, it was slowly warmed to room temperature. The reaction mixture was filtered using Celite, and the methylene chloride layer was separated from the filtrate and dried over anhydrous sodium sulphate. The solvent was distilled off under reduced pressure, and the residue purified by silica gel column chromatography (1:4 ethyl acetate: hexane) to yield 90.2 g of the title compound as an oil.
Specific rotation: $[\alpha]_D$ = -38° (C = 0.97, THF).
Optical purity (HPLC method): 88% ee.
IR spectrum: (neat) $\nu$ $cm^{-1}$:
3440, 1618, 1508, 1271.
NMR spectrum: (90 MHz, $CDCl_3$) $\delta$ ppm:
7.53-7.26 (1H, m); 6.99-6.70 (2H, m); 4.09-3.89 (2H, m); 3.29 (1H, d, J = 5.1 Hz); 2.84 (1H, d, J = 5.1 Hz); 1.81 (1H, dd, J = 8.3, 5.6 Hz).

**Example 11**

**Synthesis of 1-acetoxy-2-(2,4-difluorophenyl)-2,3-epoxypropane**

2.05 g of the 1-chloro-2-(2,4-difluorophenyl)-2,3-epoxy-propane obtained in example 8 and 0.98 g sodium acetate were suspended in 5 ml acetic acid, and heated under reflux for 1 hour. The reaction liquid was distilled off under reduced pressure, and the residue neutralised with saturated aqueous sodium hydrogen carbonate solution. It was extracted with 10 ml ethyl acetate, and after washing with water and saturated sodium chloride this was dried over anhydrous sodium sulphate. The solvent was distilled off under reduced pressure, the residue was dissolved in 20 ml 1,2-dimethoxyethane, and 400 mg sodium hydride (60% oil) were added little by little with ice-cooling. After stirring for 10 minutes, the mixture was poured into 40 ml water with ice-cooling, and extracted twice with 40 ml ethyl acetate. After washing the combined ethyl acetate layer with water and saturated sodium chloride solution, it was dried over anhydrous sodium sulphate. The solvent was distilled off under reduced pressure, and the residue distilled under reduced pressure, the fraction boiling at 137-138°C/16 mm Hg being collected to yield 1.37 g of the title compound.
B.Pt 137-138°C/16 mm Hg.
IR spectrum: (neat) $\nu$ $cm^{-1}$:
1751, 1509, 1234, 1140, 1041, 966.
NMR spectrum (90 MHz, $CDCl_3$) $\delta$ ppm:
7.53-7.26 (1H, m); 7.00-6.66 (2H, m); 4.67 (1H, d, J = 12.5 Hz); 4.15 (1H, d, J = 12.5 Hz); 3.15 (1H, d, J = 5.1 Hz); 2.85 (1H, d, J = 5.1 Hz); 2.02 (3H, s).

**Example 12**

**Synthesis of (+)-2-(2,4-difluorophenyl)-2,3-epoxypropanol and (-)-1-acetoxy-2-(2,4-difluorophenyl)-2,3-epoxypropane**

20 g of 1-acetoxy-2-(2,4-difluorophenyl)-2,3-epoxypropane obtained in example 11 were suspended in 440

ml diisopropyl ether and 4000 ml 0.1M phosphate buffer solution (pH 7.0), and 4.4 g porcine pancreatic lipase (Sigma Corp.) were added. After stirring for 3 hours at room temperature, the reaction mixture was filtered using Celite, and the filtrate was extracted twice with 3000 ml ethyl acetate. The ethyl acetate layer was washed with water and saturated sodium chloride solution and dried over anhydrous sodium sulphate. The solvent was distilled off under reduced pressure, and the residue was purified by silica gel column chromatography (1:7 ethyl acetate: hexane) to yield 8.8 g (-)-1-acetoxy-2-(2,4-difluorophenyl)-2,3-epoxypropane and 7.5 g (+)-2-(2,4-difluorophenyl-2,3-epoxypropanol, each as oils.

(-)-1-acetoxy-2-(2,4-difluorophenyl)-2,3-epoxypropane:
Optical purity (HPLC method): 98% ee.
IR spectrum (neat) ν $cm^{-1}$:
1751, 1509, 1234, 1140, 1041, 966.
NMR spectrum: (90 MHz, $CDCl_3$) δ ppm:
7.54-7.26 (1H, m); 7.00-6.66 (2H, m); 4.67 (1H, d, J = 12.5 Hz); 4.15 (1H, d, J = 12.5 Hz); 3.15 (1H, d, J = 5.1 Hz); 2.85 (1H, d, J = 5.1 Hz); 2.02 (3H, s).

(+)-2-(2,4-difluorophenyl)-2,3-epoxypropanol:
Specific rotation: $[\alpha]_D$ = +37° (C = 1.01, THF).
Optical purity (HPLC method): 87% ee.
IR spectrum: (neat) ν $cm^{-1}$:
3440, 1618, 1508, 1271.
NMR spectrum: (90 MHz, $CDCl_3$) δ ppm:
7.53-7.26 (1H, m); 6.99-6.70 (2H, m); 4.09-3.89 (2H, m); 3.29 (1H, d, J = 5.1 Hz); 2.84 (1H, d, J = 5.1 Hz); 1.81 (1H, dd, J = 8.3, 5.6 Hz).

## Example 13

### Synthesis of (-)-2-(2,4-difluorophenyl)-2,3-epoxypropanol

0.40 g of the (-)-1-acetoxy-2-(2,4-difluorophenyl)-2,3-epoxypropane obtained in example 12 were dissolved in 14 ml methanol, and 1.75 ml 1M aqueous potassium hydroxide solution were added dropwise at room temperature. After stirring for 15 minutes at room temperature, the mixture was neutralised by addition of 1M hydrochloric acid, and the methanol was distilled off under reduced pressure. After extracting twice with 10 ml ethyl acetate, this was washed with water and saturated sodium chloride solution and dried over anhydrous sodium sulphate. The solvent was distilled off under reduced pressure to yield 0.33 g of the title compound as an oil.

Specific rotation: $[\alpha]_D$ = -42° (C = 1.0, THF).
Optical purity (HPLC method): 98% ee.
IR spectrum: (neat) ν $cm^{-1}$:
3440, 1618, 1508, 1271.
NMR spectrum: (90 MHz, $CDCl_3$) δ ppm:
7.53-7.26 (1H, m); 6.99-6.70 (2H, m); 4.09-3.89 (2H, m); 3.29 (1H, d, J = 5.1 Hz); 2.84 (1H, d, J = 5.1 Hz); 1.81 (1H, dd, J = 8.3, 5.6 Hz).

## Example 14

### Synthesis of (-)-2-(2,4-difluorophenyl)-3-(1H-1,2,4-triazol-1-yl)-propan-1,2-diol

92 g of the (-)-2-(2,4-difluorophenyl)-2,3-epoxypropanol obtained in example 10 were dissolved in 750 ml tetrahydrofuran, 54 g of 1,2,4-triazole and 109 g anhydrous potassium carbonate were added, and the mixture was heated under reflux for 2 days. About 500 ml water were added to the reaction mixture, and after saturation of the aqueous layer with sodium chloride the mixture was separated and extracted twice with 800 ml ethyl acetate. The ethyl acetate layer was concentrated, and the residue was dissolved in 400 ml chloroform; 80 ml hexane were added, and after standing overnight, the crystals deposited were collected by filtration. Again, after dissolving hot in 300 ml chloroform, and standing overnight, the crystals were collected by filtration to yield 61 g of the title compound.

Specific rotation: $[\alpha]_D$ = -60° (C = 1.00, THF).
Optical purity (HPLC method): 100% ee.
M.Pt 92.1-92.8°C
IR spectrum: (KBr pellet) ν $cm^{-1}$:
3330, 3130, 1654, 1560, 1517, 1136, 1123.
NMR spectrum: (90 MHz, DMSO-$d_6$) δ ppm:

8.30 (1H, s); 7.70 (1H, s); 7.53-6.82 (3H, m); 5.77 (1H, s); 5.09 (1H, t, J = 5.6 Hz); 4.58 (2H, s); 3.67 (2H, d, J = 5.6 Hz).

About 1 mg of this compound, well powdered, was introduced into a microvial, and after addition of small amounts of methylene chloride to this, it was heated to 40°C. On addition of 0.5 ml methylene chloride, it dissolved. It was covered and allowed to stand at room temperature. On standing at least 4 days, about 10 cubic crystals, of approximately 0.1 mm side-length, were deposited. A single crystal obtained was analysed using a single crystal automatic X-ray structural analysis machine AFC-5R (Rigaku Denki Co.), and the absolute configuration was determined to be (R).

**Example 15**

**Synthesis of (+)-2-(2,4-difluorophenyl)-3-(1H-1,2,4-triazol-1-yl)-2,3-epoxypropane**

10 g of the (-)-2-(2,4-difluorophenyl)-3-(1H-1,2,4-triazol-1-yl)propan-1,2-diol obtained in example 14 were suspended in 1000 ml anhydrous ether, 27.3 ml triethylamine and 4.6 ml methanesulphonyl chloride were added at room temperature, and after stirring for 1 hour, 500 ml water were added and the mixture stirred for 1 hour more. The reaction mixture was separated, 500 ml water were added to the organic layer, the aqueous layer was adjusted to pH 4 using 1M hydrochloric acid, and the mixture was separated. The aqueous layer was washed with saturated sodium chloride solution and dried over anhydrous sodium sulphate. The solvent was distilled off under reduced pressure to yield 7.5 g of the title compound as an oil.

Specific rotation: $[\alpha]_D$ = +4° (C = 1.04, THF).
Optical purity (HPLC method): 98% ee.
IR spectrum: (neat) $\nu$ $cm^{-1}$:
1619, 1601, 1508, 1275, 1140.
NMR spectrum: (90 MHz, $CDCl_3$) $\delta$ ppm:
8.06 (1H, s); 7.87 (1H, s); 7.32-7.05 (1H, m); 6.93-6.72 (2H, m); 4.85 (1H, d, J = 14.8 Hz); 4.49 (1H, d, J = 14.8 Hz), 2.95 (1H, d, J = 4.3 Hz); 2.88 (1H, d, J = 4.3 Hz).

**Example 16**

**Synthesis of (+)-2-(2,4-difluorphenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol**

42 g of the (+)-2-(2,4-difluorophenyl)-3-(1H-1,2,4-triazol-1-yl)-2,3-epoxypropane obtained in example 15, 44.2 g of the 3-[(E)-4-(2,2,3,3-tetrafluoropropoxy)styryl]-1H-1,2,4-triazole obtained in example 6 and 80.6 g anhydrous potassium carbonate were suspended in 268 ml dimethylformamide, and heated at 90°C with stirring for 1 hour. The reaction mixture was poured into 800 ml 1M hydrochloric acid, extracted with ethyl acetate, and after washing with water and saturated sodium chloride solution was dried over anhydrous sodium sulphate. The solvent was distilled off under reduced pressure, and the residue purified by silica gel column chromatography (elution with ethyl acetate containing 2% methanol, after elution of the low polarity compounds with 6:1 ethyl acetate:hexane); the fractions containing the desired compound were concentrated, and crystallised by addition of ether to the residue. This was dissolved in 700 ml ethyl acetate with heating, 2400 ml hexane were added at 50°C, and after standing 1.5 hours at room temperature the crystals deposited were collected by filtration. These crystals were dissolved in 100 ml ethyl acetate, 300 ml hexane were added at 50°C, and after standing for 1 hour at room temperature the crystals deposited were collected by filtration to yield 18 g of the title compound.

Specific rotation: $[\alpha]_D$ = +50.7° (C = 1.01, THF).
Optical purity (HPLC method): 99.5% ee.
Chemical purity (HPLC method): 99.8%.
M.Pt. 92.0-93.4°C.
IR spectrum: (KBr pellet) $\nu$ $cm^{-1}$:
3130, 1618, 1606, 1515, 1500, 1114.
NMR spectrum: (90 MHz, DMSO-$d_6$) $\delta$ ppm:
8.32 (1H, s); 8.28 (1H, s); 7.77 (1H, s); 7.59 (2H, d, J = 8.6 Hz);
7.34 (1H, d, J = 16.2 Hz); 7.04; (2H, d, J = 8.6 Hz); 6.90 (1H, d, J = 16.2 Hz); 7.35-6.86 (3H, m); 6.66 (1H, tt, J = 51.8, 5.6 Hz); 6.37 (1H, s); 4.88-4.55 (6H, m).

**Example 17**

**Synthesis of (+)-2-(2,4-difluorphenyl)-3-[3-[(E)-4-(2,2,3,3-tetrafluoropropoxy)styryl]-1H-1,2,4-triazol-1-**

**yl]propan-1,2-diol**

0.45 g of the (+)-2-(2,4-difluorophenyl)-2,3-epoxypropanol obtained in example 12 and 0.60 g of the 3-[(E)-4-(2,2,3,3-tetrafluoropropoxy)styryl]-1H-1,2,4-triazole obtained in example 6 were dissolved in 20 ml dimethylformamide, 0.28 g potassium carbonate were added and the mixture was stirred for 3 hours at 80-90°C. After allowing to cool, 50 ml water were added to the reaction mixture, and it was extracted 3 times with 50 ml ethyl acetate. The combined ethyl acetate layers were washed with water and saturated sodium chloride solution and dried over anhydrous sodium sulphate. The solvent was distilled off under reduced pressure and the residue purified by silica gel column chromatography (1:1-1.3 ethyl acetate:hexane), to yield 0.42 g of the title compound.

Specific rotation: $[\alpha]_D$ = +57.4° (C = 1.02, THF).
Optical purity (HPLC method): 88% ee.
M.Pt. 119.8-120.8°C.
IR spectrum: (KBr pellet) $\nu$ $cm^{-1}$:
3446, 1607, 1516, 1499, 1261, 1126.
NMR spectum: (90 MHz, $CDCl_3$) $\delta$ ppm:
7.86 (1H,s); 7.68-7.40 (4H, m); 6.96-6.66 (5H, m); 6.06 (1H, tt, J = 53.1, 5.0 Hz); 4.71 (2H, s); 4.36 (2H, t, J = 11.7 Hz); 4.03 (1H, d, J = 11.7 Hz); 3.75 (1H, d, J = 11.5 Hz).

**Example 18**

**Synthesis of (-)-2-(2,4-difluorophenyl)-1-[3-[(E)-4-(2,2,3,3-tetrafluoropropoxy)styryl]-1H-1,2,4-triazol-1-yl]-2,3-epoxypropane**

300 mg of the (+)-2-(2,4-difluorophenyl-3-[3-[(E)-4-(2,2,3,3-tetrafluoropropoxy)styryl]-1H-1,2,4-triazol-1-yl]propan-1,2-d iol obtained in example 17 were dissolved in 30 ml anhydrous ether, and 0.07 ml methanesulphonyl chloride and 0.43 ml triethylamine were added. After stirring for 30 minutes, the reaction mixture was concentrated under reduced pressure, the residue dissolved in 25 ml dimethylformamide, and 50 mg sodium hydride (60% oil) were added. After stirring for 4 hours at room temperature, 50 ml water were added to the reaction mixture, and it was extracted 3 times with 40 ml ethyl acetate. The combined ethyl acetate layers were washed with water and saturated sodium chloride solution and dried over anhydrous sodium sulphate. The solvent was distilled off under reduced pressure and the residue purified by silica gel column chromatography (2:3 ethyl acetate:hexane) to yield 187 mg of the title compound.

Specific rotation: $[\alpha]_D$ = -11.8° (C = 0.765, THF).
Optical purity (HPLC method): 87% ee.
M.Pt. 129.8-132.3°C.
IR spectrum: (KBr pellet) $\nu$ $cm^{-1}$:
1619, 1509, 1271, 1214, 1138, 1101.
NMR spectrum: (90 MHz, $CDCl_3$) $\delta$ ppm:
7.99 (1H, s); 7.60-6.73 (9H, m); 6.07 (1H, tt, J = 53.1, 4.9 Hz); 4.80 (1H, d, J = 15.2 Hz); 4.48 (1H, d, J = 15.2 Hz); 4.36 (2H, t, J = 11.7 Hz); 2.98 (1H, d, J = 4.8 Hz); 2.88 (1H, d, J = 4.8 Hz).

**Example 19**

**Synthesis of (+)-2-(2,4-difluorophenyl)-1-[3-[(E)-4-(2,2,3,3-tetrafluoropropoxy)styryl]-1H-1,2,4-triazol-1-yl]-3-(1E-1,2,4-triazol-1-yl)propan-2-ol**

22 mg 1,2,4-triazole were dissolved in 3 ml dimethylformamide and 14 mg sodium hydride (60% oil) were added. After the foaming had subsided, a solution of 150 mg of the (-)-2-(2,4-difluorophenyl)-1-[3-[(E)-4-(2,2,3,3-tetrafluoropropoxy) styryl]-1H-1,2,4-triazol-1-yl]-2,3-epoxypropane obtained in example 18 dissolved in 3.5 ml dimethylformamide was added dropwise. After stirring 16 hours at room temperature, a further 4 mg sodium hydride (60% oil) and 7 mg 1,2,4-triazole were added. After stirring 4 hours more at room temperature, 80 ml water were added, and the mixture was extracted twice with 80 ml ethyl acetate. The combined ethyl acetate layers were washed with water and saturated sodium chloride solution and dried over anhydrous sodium sulphate. The solvent was distilled off under reduced pressure, and the residue purified by silica gel column chromatography (ethyl acetate). The fractions containing the desired compound were concentrated, the residue was dissolved in 2 ml ethyl acetate with heating, and after addition of 9 ml hexane at 50°C the mixture was allowed to stand at room temperature; the crystals deposited were collected by filtration to yield 60 mg of the title compound.

Optical purity (HPLC method): 96% ee.
M.Pt 92.0-93.4°C.

IR spectrum (KBr pellet) ν $cm^{-1}$:
3130, 1618, 1606, 1515, 1500, 1114.
NMR spectrum (90 MHz, DMSO-$d_6$) δ ppm:
8.32 (1H, s); 8.28 (1H, s); 7.77 (1H, s); 7.59 (2H, d, J = 8.6 Hz);
7.34 (1H, d, J = 16.2 Hz); 7.04 (2H, d, J = 8.6 Hz); 6.90 (1H, d, J = 16.2 Hz); 6.86-7.42 (3H, m); 6.66 (1H, tt, J = 51.8, 5.6 Hz); 6.37 (1H, s); 4.88-4.45 (6H, m).

**Example 20**

**Synthesis of (+)-2-(2,4-difluorophenyl)-1-[3-[(E)-4-(2,2,3,3-tetrafluoropropoxystyryl]-1H-1,2,4-triazol-1-yl]-3-(1H-1,2,4-triazol-1-yl)propan-2-ol monosulphate**

100 mg of the (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol obtained in example 16 were dissolved in 1 ml ethanol, a solution of 18.2 mg concentrated sulphuric acid in 0.5 ml ethanol was added, and the mixture was allowed to stand for 2 hours. The crystals deposited were collected by filtration and after air drying they were dissolved in 3 ml ethanol with heating. After standing for 16 hours at room temperature, the crystals deposited were collected by filtration and air dried. The crystals obtained were suspended in 2 ml acetone, and after ultrasonic treatment for 2 hours, were collected by filtration, and dried under reduced pressure to yield 55 mg of the title compound.
Elemental analysis: $C_{24}H_{20}N_6O_2F_6.H_2SO_4$
Found: C(44.82%) H(3.43%), N(12.75%)
Calc: C(44.29%) H(3.48%), N(13.20%)
M.Pt 189-193°C.
IR spectrum (KBr pellet) ν $cm^{-1}$:
1606, 1514, 1262, 1202, 1178, 1124, 620.
NMR spectrum (90 MHz, DMSO-$d_6$) δ ppm:
8.52 (1H, s); 8.37 (1H, s); 7.93 (1H, s); 7.75-6.81 (9H, m); 6.03 (1H, tt, J = 51.8, 5,6 Hz); 4.85-4.46 (6H, m).

**Example 21**

**Optical isomerisation of 2-(2,4-difluorophenyl)-2,3-epoxypropanol**

**Stage 1**

**Synthesis of (+)-1-chloro-2-(2,4-difluorophenyl)-2,3-epoxypropane**

1.07 g of the (+)-2-(2,4-difluorophenyl)-2,3-epoxypropanol obtained in example 12 and 1.7 g triphenylphosphine were dissolved in 18 ml carbon tetrachloride, and heated 7 hours under reflux. After allowing to cool, 20 ml water were added and the mixture was extracted twice with 20 ml methylene chloride. The combined organic layers were washed with water and saturated sodium chloride solution and dried over anhydrous sodium sulphate. The solvent was distilled off under reduced pressure, and the residue purified by silica gel column chromatography (3:97 ethyl acetate:hexane) to give 0.71 g of the title compound as an oil.
IR spectrum (neat) ν $cm^{-1}$.
1619, 1602, 1508, 1425, 1272.
NMR spectrum (90 MHz, $CDCl_3$) δ ppm:
7.57-7.31 (1H, m); 6.98-6.71 (2H, m); 4.09 (1H, d, J = 11.9 Hz); 3.68 (1H, d, J = 11.9 Hz); 3.20 (1H, d, J = 4.8 Hz); 2.93 (1H, d, J = 4.8 Hz).

**Stage 2**

**Synthesis of (-)-1-acetoxy-2-(2,4-difluorophenyl)-2,3-epoxypropane**

Using 0.70 g of the (+)-1-chloro-2-(2,4-difluorophenyl)-2,3-epoxypropane obtained in stage 1, by the same method as in example 11, 0.55 g of the title compound were obtained as an oil.
IR spectrum (neat) ν $cm^{-1}$:
1751, 1509, 1234, 1140, 1041, 966.
NMR spectrum (90 MHz, $CDCl_3$) δ ppm:
7.54-7.26 (1H, m); 7.00-6.66 (2H, m); 4.67 (1H, d, J -12.5 Hz); 4.15 (1H, d, J =12.5 Hz); 3.15 (1H, d, J = 5.1 Hz); 2.85 (1H, d, J = 5.1 Hz); 2.02 (3H, s).

**Stage 3**

**Synthesis of (-)-2-(2,4-difluorophenyl)-2,3-epoxypropanol**

Using 0.40 g of the (-)-1-acetoxy-2-(2,4-difluorophenyl)-2,3-epoxypropane obtained in stage 2, by the same method as in example 13, 0.33 g of the title compound were obtained as an oil.
Optical purity (HPLC method): 98% ee.
IR spectrum (neat) ν $cm^{-1}$:
3440, 1618, 1508, 1271.
NMR spectrum (90 MHz, $CDCl_3$ δ ppm:
7.53-7.26 (1H, m); 6.99-6.70 (2H, m); 4.09-3.89 (2H, m); 3.29 (1H, d, J = 5.1 Hz); 2.84 (1H, d, J = 5.1 Hz); 1.81 (1H, dd, J = 8.3, 5.6 Hz).

**Example 22**

**Synthesis of 1-(2,4-difluorophenyl)cyclopropanol**

2.5 g of the 1,3-dichloro-2-(2,4-difluorophenyl)propan-2-ol obtained in example 7 were dissolved in 20 ml anhydrous ether, and an ether solution (40 ml) of ethyl-magnesium bromide prepared from 7.91 g ethyl bromide and 1.84 g magnesium, and an ether solution (10 ml) of ferric chloride were added dropwise simultaneously over a period of 2 hours with ice-cooling and vigorous stirring. After stirring 1 hour at room temperature, the reaction mixture was poured into a mixture of 80 g ice and 30 ml 2M hydrochloric acid saturated with ammonium chloride, and the ether layer was separated. After extraction of the aqueous layer with 50 ml ether, the ether layers were combined, and, after washing with saturated sodium chloride solution, dried over anhydrous magnesium sulphate. The solvent was distilled off under reduced pressure and the residue purified by silica gel column chromatography (1:9 ethyl acetate:hexane) to yield 0.99 g of the title compound as an oil.
IR spectrum (neat) ν $cm^{-1}$:
1617, 1604, 1508, 1234, 1140, 1115, 969, 851.
NMR spectrum (90 MHz, $CDCl_3$) δ ppm:
7.51-6.69 (3H, m); 2.53 (1H, d, J = 1.7 Hz); 1.18-0.89 (4H, m).

**Example 23**

**Synthesis of 1-(2,4-difluorophenyl)-1-(methanesulphonyloxy)cyclopropane**

After addition, with ice-cooling, of 0.72 g methanesulphonyl chloride to a solution of 0.97 g of the 1-(2,4-difluorophenyl)-cyclopropanol obtained in example 22 and 0.63 g triethylamine in 8 ml ether, the mixture was stirred at room temperature for 1 hour. The reaction mixture was poured into 15 ml 1M hydrochloric acid, and separated after thorough stirring. The ether layer was washed with saturated sodium chloride solution and after drying over anhydrous magnesium sulphate it was concentrated under reduced pressure to yield 1.26 g of the title compound as an oil.
IR spectrum (neat) ν $cm^{-1}$:
3089, 2941, 1509, 1363, 1171.
NMR spectrum (90 MHz, $CDCl_3$) δ ppm:
7.62-6.74 (3H, m); 2.68 (3H, s); 1.76-1.08 (4H, m).

**Example 24**

**Synthesis of 2-(2,4-difluorophenyl)allyl alcohol**

1.0 g of the 1-(2,4-difluorophenyl)-1-(methanesulphonyloxy)cyclopropane obtained in example 23 and 0.444 g potassium carbonate were added to a mixed solvent of 10 ml water and 10 ml acetone, and stirred for 10 hours at 50°C. The reaction mixture was extracted with 400 ml ether, and after washing of the organic layer with saturated sodium chloride solution it was dried over anhydrous sodium sulphate. The solvent was distilled off under reduced pressure and the residue purified by silica gel column chromatography (1:6 ethyl acetate:hexane) to yield 0.39 g of the title compound as an oil.
IR spectrum (neat) ν $cm^{-1}$:
3340, 1617, 1506, 1420, 1267.
NMR spectrum (90 MHz, $CDCl_3$) δ ppm:
7.44-7.18 (1H, m); 6.96-6.69 (2H, m); 5.51 (1H, m); 5.35 (1H, bs), 4.46 (2H, m); 1.71-1.64 (1H, m).

**Example 25**

**Synthesis of 2-(2,4-difluorophenyl)-1,2-epoxypropane**

8.5 g sodium hydride (60% oil) were suspended in 170 ml dimethyl sulphoxide and, after gradual addition of 42.8 g trimethylsulphoxonium iodide with ice-cooling, a solution of 27.6 g 2,4-difluoroacetophenone in 140 ml dimethyl sulphoxide was added dropwise at 50°C. After stirring 4 hours at 50°C, the reaction mixture was poured into 1 litre ice-water, and extracted with 800 ml ether. After extracting the aqueous layer twice more with 500 ml ether, the combined ether layers were washed 3 times with water, then once with saturated sodium chloride solution, and then dried over anhydrous magnesium sulphate. The solvent was distilled off under reduced pressure, and the residue distilled under reduced pressure, the 90-92°C/40 mm Hg fraction being collected to yield 17.2 g of the title compound.
B.Pt. 90-92°C/40 mm Hg
IR spectrum (neat) $\nu$ $cm^{-1}$:
2985, 1617, 1604, 1507, 1424, 1271, 1140, 967, 851.
NMR spectrum (90 MHz, $CDCl_3$) $\delta$ ppm:
7.42-6.70 (3H, m); 2.95 (1H, d, J = 5.3 Hz); 2.78 (1H, d, J = 5.3 Hz); 1.65 (3H, s).

**Example 26**

**Synthesis of 2-(2,4-difluorophenyl)allyl alcohol**

2.18 g of the 2-(2,4-difluorophenyl)-1,2-epoxypropane obtained in example 25 were dissolved in 25 ml acetonitrile, and 6 ml 1,8-diazabicyclo[5.4.0]-undec-7-ene were added. After dropwise addition of 4 ml trimethylsilyl iodide to this, the mixture was heated under reflux for 20 hours. The reaction mixture was poured into water, extracted 3 times with 50 ml ether, and after successive washing of the organic layer with 1 M hydrochloric acid and saturated sodium chloride solution, it was dried over anhydrous magnesium sulphate. The solvent was distilled off under reduced pressure and the residue purified by silica gel column chromatography (1:4 ethyl acetate:hexane) to yield 0.4 g of the title compound as an oil.
IR spectrum (neat) $\nu$ $cm^{-1}$:
3340, 1617, 1506, 1420, 1267.
NMR spectrum (90 MHz, $CDCl_3$) $\delta$ ppm:
7.44-7.18 (1H, m); 6.96-6.69 (2H, m); 5.51 (1H, m); 5.35 (1H, bs), 4.46 (2H, m); 1.71-1.64 (1H, m).

Next, we show examples of formulations containing (+)-2-(2,4-difluorophenyl)-1-[3-[(E)-4-(2,2,3,3-tetrafluoropropoxy)-styryl]-1H-1,2,4-triazol-1-yl]-3-(1H-1,2,4-triazol-1-yl)propan-2-ol (compound (+)-I) or salts thereof, but the present invention is not limited by the examples below.

**Example A** **Tablets**

| | |
|---|---|
| Compound (+)-I | 100 g |
| Polyethylene glycol 6000 | 100 g |
| Sodium lauryl sulphate | 15 g |
| Corn starch | 30 g |
| Lactose | 250 g |
| Magnesium stearate | 5 g |

After weighing out the above ingredients, the polyethylene glycol 6000 was heated to 70-80°C, and the compound of the invention, the sodium lauryl sulphate, corn starch and lactose were added to this, and cooled after mixing. The solidified mixture was placed in a crusher and pulverised. After mixing with the magnesium stearate, this granulate was made into 250 mg tablets by compression tabletting.

**Example B** **Capsules**

| | |
|---|---|
| Compound (+)-I monosulfate | 100 g |
| Lactose | 250 g |
| Corn starch | 50 g |
| Microcrystalline cellulose | 95 g |
| Magnesium stearate | 5 g |

After weighing out each of the above ingredients, all 4 except the magnesium stearate uniformly mixed. The magnesium stearate was added, and then mixing was resumed for a few minutes. The capsules were made by filling 250 mg portions of the mixture into No. 1 hard capsules, using an encapsulator.

**Example C** **Suppositories**

| | |
|---|---|
| Compound (+)-I | 50 g |
| Polyethylene glycol 1500 | 200 g |
| Polyethylene glycol 4000 | 750 g |

After making the compound (+)-I of the invention into a fine powder by grinding in a mortar, it was made into 1 g suppositories by the solution melt method.

## Claims

1. (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl) propan-2-ol shown in the formula ((+)-I)

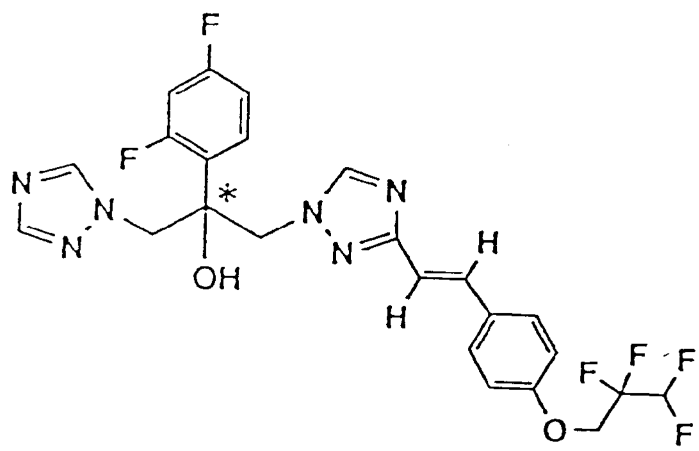

((+)-I)

(where * indicates an optically active centre), pharmacologically acceptable salts thereof, solvates thereof and solvates of salts thereof.

2. (-)- or (+)-2-(2,4-difluorophenyl)propane derivatives shown in the formula (II)

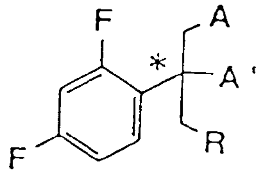

(II)

(where * indicates an optically active centre, point A and A′ together are an oxygen atom, or A′ is a hydroxy group and A is a hydroxy group, methanesulfonyloxy group or p-toluenesulfonyloxy group, and R is a hydroxy group, acetoxy group, 1H-1,2,4-triazol-1-yl group or 3-[(E)-4-(2,2,3,3-tetrafluoropropoxy)styryl]-1H-1,2,4-triazol-1-yl group, providing that both A and R are not simultaneously hydroxy groups).

3. Compounds according to Claim 2, in which A and A′ together are an oxygen atom.

4. Compounds according to Claim 2, in which A and A′ are both hydroxy groups.

5. Compounds according to Claim 3, in which R is a hydroxy group or an acetoxy group.

6. Compounds according to Claim 3, in which R is a 1H-1,2,4-triazol-1-yl group.

7. Compounds according to Claim 3, in which R is a 3-[(E)-4-(2,2,3,3-tetrafluoropropoxy)styryl]-1H-1,2,4-triazol-1-yl group.

8. Compounds according to Claim 4, in which R is a 1H-1,2,4-triazol-1-yl group.

9. Compounds according to Claim 4, in which R is a 3-[(E)-4-(2,2,3,3-tetrafluoropropoxy)styryl]-1H-1,2,4-triazol-1-yl group.

10. Method for preparation of (+)-2-(2,4-difluorophenyl)-1-[3-[(E)-4-(2,2,3,3-tetrafluoropropoxy)styryl]-1H-1,2,4-triazol-1-yl] -3-(1H-1,2,4-triazol-1-yl)propan-2-ol shown in formula ((+)-I)

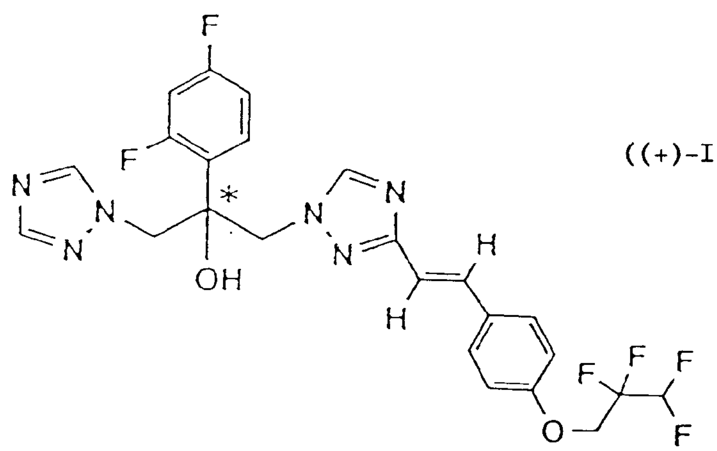

((+)-I)

where * indicates an optically active centre, pharmacologically acceptable salts thereof, solvates thereof and solvates of salts thereof, characterised in that 1,2,4-triazole is reacted with (-)-2-(2,4-difluorophenyl)-2,3-epoxypropanol shown by the formula ((-)-VII)

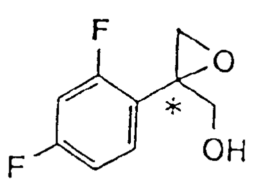

((-)-VII)

where * indicates an optically active centre, in the presence of base, next the epoxide is made under basic conditions after activation of the primary hydroxy group with methanesulphonyl chloride, and then reacted in presence of base with 3-[(E)-4-(2,2,3,3-tetrafluoropropoxy)styryl]-1H-1,2,4-triazole shown by the formula (XII)

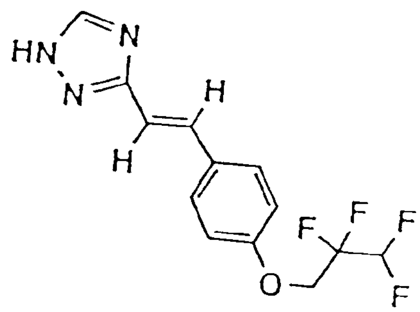

(XII)

11. Method for preparation of (+)-2-(2,4-difluorophenyl)-1-[3-[(E)-4-(2,2,3,3-tetrafluoropropoxy)styryl]-1H-1,2,4-triazol-1-yl] -3-(1H-1,2,4-triazol-1-yl)propan-2-ol shown in formula ((+)-I)

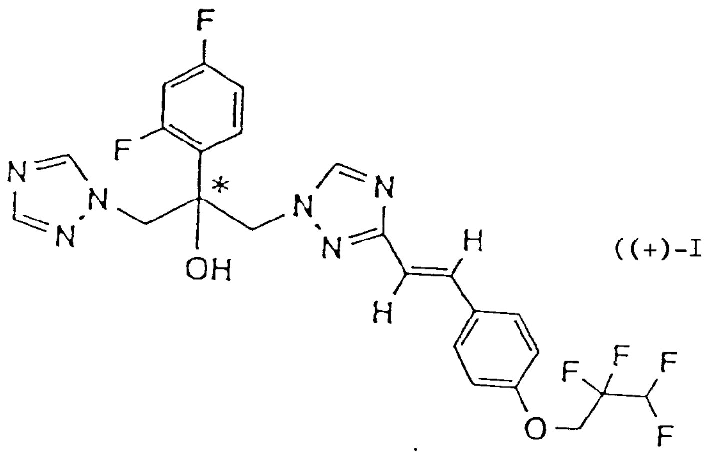

((+)-I)

where * indicates an optically active centre, pharmacologically acceptable salts thereof, solvates thereof and solvates of salts thereof, characterised in that 3-[(E)-4-(2,2,3,3-tetrafluoropropoxy)styryl]-1H-1,2,4-triazole shown by the formula (XII)

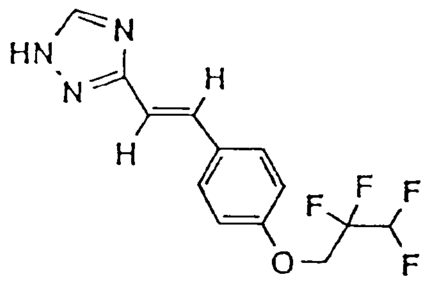

(XII)

is reacted with (+)-2-(2,4-difluorophenyl)-2,3-epoxypropanol shown by the formula ((+)-VII)

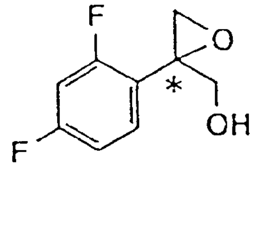

((+)-VII)

where * indicates an optically active centre, in the presence of base, next the epoxide is made under basic conditions after activation of the primary hydroxy group with methanesulphonyl chloride, and then reacted with 1,2,4-triazole in presence of base.

**12.** Method for the preparation of (-)-2-(2,4-difluorophenyl)-2,3-epoxypropanol shown by the formula ((-)-VII)

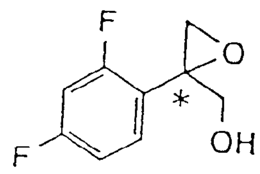

((-)-VII)

where * indicates an optically active centre, characterised in that 2-(2,4-difluorophenyl)allyl alcohol shown by the formula (VI)

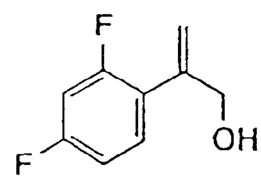

(VI)

is subjected to asymmetrical oxidation using t-butyl hydroperoxide in the presence of titanium tetraisopropoxide and diethyl (+)-tartrate.

**13.** Method for the preparation of (+)-2-(2,4-difluorophenyl)-2,3-epoxypropanol shown by the formula ((+)-VII)

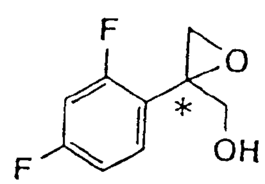

((+)-VII)

where * indicates an optically active centre, characterised in that 2-(2,4-difluorophenyl)allyl alcohol shown by the formula (VI)

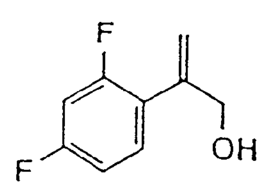

(VI)

is subjected to asymmetrical oxidation using t-butyl hydroperoxide in the presence of titanium tetraisopropoxide and diethyl (-)-tartrate.

**14.** Method for the preparation of optically active 2-(2,4-difluorophenyl)-2,3-epoxypropanols characterised in that (+)-2-(2,4-difluorophenyl-2,3-epoxypropanol shown by the formula ((+)-VII)

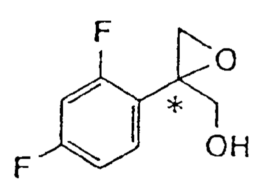

((+)-VII)

where * indicates optically active centre, is obtained by hydrolysis by the action of lipase on 1-acetoxy-2-(2,4-difluorophenyl)-2,3-epoxypropane shown by the formula ((±)-IX)

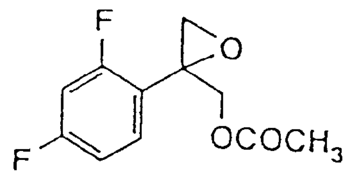

((±)-IX)

and (-)-2-(2,4-difluorophenyl)-2,3-epoxypropanol shown by the formula ((-)VII)

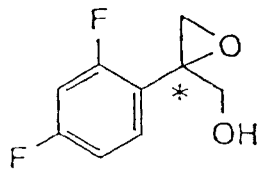

((-)-VII)

where * indicates an optically active centre, is obtained by hydrolysis of the recovered (-)-1-acetoxy-2-(2,4-difluorophenyl)-2,3-epoxypropane shown by the formula ((-)-IX)

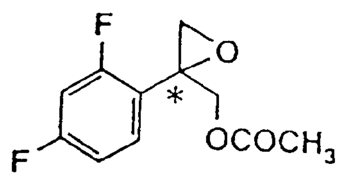

((-)-IX)

where * indicates an optically active centre, by reaction with alkali.

**15.** Antifungal agents containing as active ingredient at least one of (+)-2-(2,4-difluorophenyl)-1-[3-[(E)-4-(2,2,3,3-tetrafluoropropoxy)styryl]-1H-1,2,4-triazol-1-yl]-3-(1H-1,2,4-triazol-1-yl)propan-2-ol shown by the formula ((+)-I)

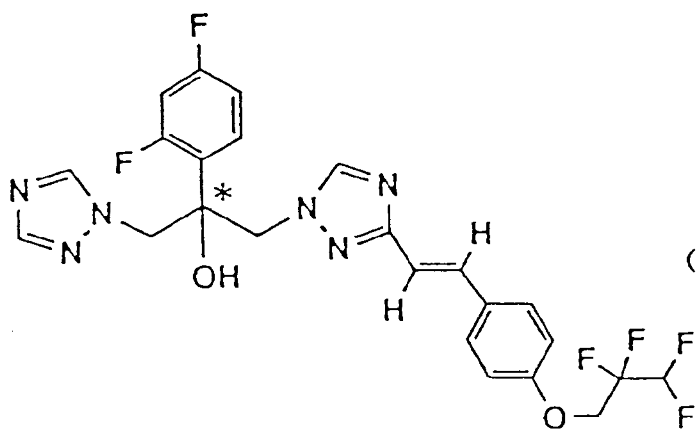

((+)-I)

wherein * indicates an optically active centre, pharmacologically acceptable salts thereof, solvates thereof and/or solvates of salts thereof.